# EUROPÄISCHE PATENTSCHRIFT

(11) **EP 2 531 505 B1**
(45) Veröffentlichungstag und Bekanntmachung des Hinweises auf die Patenterteilung: **15.03.2017**
(21) Anmeldenummer: 11701766.5
(22) Anmeldetag: 28.01.2011
(51) Int. Cl.: C07D 471/08, C07F 13/00, C07F 15/02

(54) **VERFAHREN ZUR HERSTELLUNG VON 3,7-DIAZA-BICYCLO[3.3.1]NONAN-METALLKOMPLEXEN**
PROCESS FOR THE PREPARATION OF 3,7-DIAZA-BICYCLO[3.3.1]NONAN-METAL-COMPLEX-SOLUTIONS
PROCÉDÉ DE PRÉPARATION DE SOLUTIONS DE COMPLEXE DE METAL ET DE 3,7-DIAZA-BICYCLO[3.3.1]NONAN

(30) Priorität: 06.02.2010 DE 102010007059
(43) Veröffentlichungstag der Anmeldung: 12.12.2012
(73) Patentinhaber: WeylChem Switzerland AG, 4132 Muttenz (CH)
(72) Erfinder: REINHARDT, Gerd, 65779 Kelkheim (DE); LADWIG, Miriam, 63128 Dietzenbach (DE); BEST, Michael, 65812 Bad Soden (DE); SCHNEIDER, Thorsten, 65589 Hadamar (DE); SPICKER, Ernst, 60596 Frankfurt am Main (DE)
(74) Vertreter: Ackermann, Joachim
(86) Internationale Anmeldenummer: PCT/EP2011/000404
(87) Internationale Veröffentlichungsnummer: WO 2011/095308

(56) Entgegenhaltungen:
- WO-A1-01/16271
- DE-A1-102008 064 009

## Beschreibung

Die Erfindung betrifft ein Verfahren zur Herstellung von Eisen- oder Mangankomplexen mit 3,7-Diaza-bicyclo[3.3.1]nonan-Liganden in heterogenen, wässrigen Systemen.

3,7-Diaza-bicyclo[3.3.1]nonan-Verbindungen stellen interessante Verbindungen für verschiedene Anwendungen dar. Unter anderem sind Übergangsmetallkomplexe, die einen Liganden gemäß Formel (1) enthalten, sehr effektive Katalysatoren, die in Kombination mit Peroxiden zur Bleiche farbiger Anschmutzungen in Wasch- und Reinigungsmitteln eingesetzt werden können. Beispiele hierfür finden sich in WO 00/60045 und EP 1 678 286. Für diese Anwendung sind hohe Produktreinheiten erforderlich, da Spuren freier Metallionen zu unerwünschten Nebenreaktionen und damit zur Schädigung des Waschgutes beitragen können. Einige dieser Komplexe sind aber auch überaus wirksam in Gegenwart von Luftsauerstoff und ermöglichen eine Bleiche ölhaltiger Anschmutzungen unter Verzicht auf die Verwendung der ansonsten üblichen Wasserstoffperoxide oder anorganischen Persalze. Beispiele hierfür werden u. a. in WO 03/104234 beschrieben.

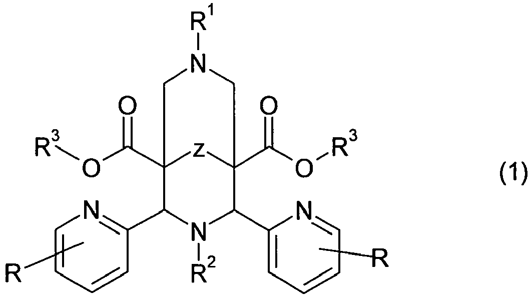

Aufgrund ihres Wirkmechanismus mit Sauerstoff hat sich in jüngster Zeit ein weiteres Anwendungsgebiet für diese Substanzklasse erschlossen. So wird in WO 2008/003652 die Verwendung solcher Übergangsmetallkomplexe als Katalysatoren für die Trocknung von Alkyd-basierenden Farben und Lacken beschrieben. Hierbei dienen sie als umweltfreundliche Alternative zu Cobalthaltigen Fettsäurederivaten, die im Verdacht stehen, Krebs zu erzeugen.

Liganden der Formel (1) und deren Metallkomplexe sind in der Literatur eingehend beschrieben. Die Ligandsynthese wird z. B. in WO 2006/133869 beschrieben, während in WO 02/48301, in Inorg. Chimica Acta, 337 (2002) 407 - 419 und in Eur. J. Org. Chem. (2008) 1019 - 1030 Komplexierungsreaktionen beschrieben werden.

Die bekannten Komplexsynthesen erfolgen durch Umsetzung des jeweiligen Liganden der Formel (1) mit einem Metallsalz in homogener Lösung. Dabei werden sowohl der Ligand als auch das Metallsalz separat in unterschiedlichen organischen Lösemitteln gelöst und anschließend die Komplexbildung in homogener Lösung durchgeführt. Hierbei wird unter Argon oder Stickstoff und wasserfreien Bedingungen gearbeitet. Da auch die gebildeten Metallkomplexe gute Löslichkeit im Lösemittelgemisch aufweisen, muss zur Isolierung der Komplexe ein weiteres Lösemittel eingesetzt werden, um das Produkt in kristallener Form isolieren zu können. Die Ausbeuten sind dabei nur moderat und liegen zwischen 40 und 70 %.

Die beschriebenen Syntheseverfahren erfordern für die Isolierung des Endproduktes hohe Lösemittelzusätze zur Reinigung, wie z. B. Methanol, Essigester, Aceton oder Dichlormethan. Die Wahl der Lösungsmittel und die streng wasserfreien Bedingungen (wasserfreie Lösungsmittel, Argon- oder Stickstoff-Überlagerung der Reaktion) führen zu Problemen und Verteuerung bei der großtechnischen Umsetzung. Es bestand daher der Bedarf an einem verbesserten großtechnisch durchführbaren Verfahren zur Herstellung solcher Komplexe.

Überraschenderweise wurde nun gefunden, dass Eisen- und Mangankomplexe der Formel (2) in einer heterogenen Reaktion in Wasser hergestellt werden können, obwohl Liganden gemäß Formel (1) in Wasser praktisch unlöslich sind. Weiterhin gelingt eine Verbesserung der Raum-Zeit-Ausbeute unter Einhaltung bestimmter Reaktionsbedingungen, wobei man in hohen Ausbeuten und Reinheiten zu den gewünschten Metallkomplexen gelangt.

Gegenstand der vorliegenden Erfindung ist daher ein Verfahren zur Herstellung eines oder mehrerer Metallkomplexe der allgemeinen Formel (2)

[MₐLₓXₙ]Yₘ (2)

wobei
- M: ein Metall aus der Gruppe Mn(II), Mn(III), Mn(IV), Fe(II), Fe(III) oder Fe(IV) ist,
- X: eine koordinierende Verbindung ausgewählt aus mono-, bi- oder trigeladenen Anionen oder neutralen Molekülen ist, die an ein Metall mono-, bi- oder tridentat koordinieren können, vorzugsweise OH⁻, NO₃⁻, NO, S²⁻, R^{a}S⁻, PO₄³⁻, H₂O, CO₃²⁻, R^{b}OH, Cl⁻, Br⁻, CN-, ClO₄⁻, R^{a}COO⁻ oder SO₄²⁻, wobei R^{a} H oder C₁-C₄ Alkyl und R^{b} C₁-C₄ Alkyl ist,
- Y: ein nicht koordinierendes Gegenion darstellt, welches den Ladungsausgleich des Komplexes gewährleistet, vorzugsweise R^{c}SO₄⁻, SO₄²⁻, NO₃⁻, Cl⁻, Br⁻, I⁻, ClO₄⁻, BF₄⁻, PF₆⁻, oder R^{c}SO₃⁻, wobei R^{c} H oder C₁-C₄ Alkyl ist,
- a: eine Zahl von 1 bis 2 ist,
- x: eine Zahl von 1 bis 2 ist,
- n: eine Zahl von 0 bis 4 ist,
- m: eine Zahl von 0 bis 8 ist, und
- L: einen Liganden der allgemeinen Formel (1) oder seine protonierte oder deprotonierte Form 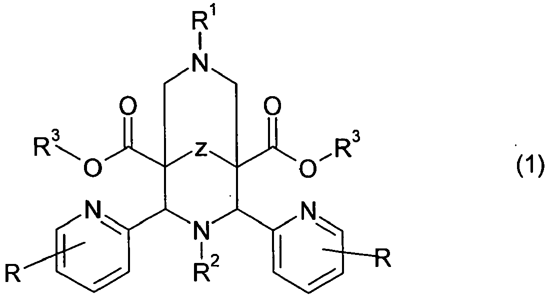
darstellt, wobei
- R: Wasserstoff, Hydroxyl oder C₁-C₄ Alkyl ist;
- R¹: C₁-C₄ Alkyl, C₆-C₁₀ Aryl, Pyridinyl-C₁-C₄-alkyl oder (CH₂)ₖN(C₁-C₄-alkyl)₂ ist;
- R²: C₁-C₂₀ Alkyl, C₆-C₁₀ Aryl oder Pyridinyl-C₁-C₄-alkyl ist;
- R³: C₁-C₄ Alkyl ist;
- z: C=O oder C(OH)₂ ist und
- k: eine Zahl von 1 bis 6 bedeutet,
dadurch gekennzeichnet, dass die Umsetzung eines oder mehrerer Liganden der Formel (1) mit einem Eisen- oder Mangansalz, insbesondere Eisen(II)-chlorid, in heterogener Reaktion in Wasser durchgeführt wird, wobei die Reaktion in einem Temperaturbereich von 35 bis 50 °C, vorzugsweise von 40 bis 46 °C, durchgeführt wird und der pH-Wert des Reaktionsgemisches nach Zugabe des Eisen- oder Mangansalzes, insbesondere des Eisen(II)-chlorids, von 1 bis 3 und vorzugsweise von 1,5 bis 2,5 beträgt.

Besonders bevorzugt werden mit dem erfindungsgemäßen Verfahren ein oder mehrere Komplexe der Formel [FeLCl]Cl, [FeL(SO₄)], [MnLCl]Cl, [MnL(SO₄)], [FeLCl]PF₆, [FeL(H₂O)][PF₆]₂ oder [FeL(H₂O)][BF₄]₂ hergestellt,
wobei L insbesondere ausgewählt ist aus der Gruppe bestehend aus
2,4-Di-(2-pyridyl)-3-methyl-7-(pyridin-2-ylmethyl)-3,7-diazabicyclo[3.3.1]nonan-9-on-1,5-dimethyl-dicarboxylat (N2Py3o),
2,4-Di-(2-pyridyl)-3-(pyridin-2-ylmethyl)-7-methyl-3,7-diazabicyclo[3.3.1]nonan-9-on-1,5-dimethyl-dicarboxylat (N2Py3u),
2,4-Di-(2-pyridyl)-3-methyl-7-(pyridin-2-ylmethyl)-3,7-diazabicyclo[3.3.1]nonan-9-on-1,5-diethyl-dicarboxylat,
2,4-Di-(2-pyridyl)-3,7-bis-(pyridin-2-ylmethyl)-3,7-diazabicyclo[3.3.1]nonan-9-on-1,5-dimethyl-dicarboxylat (N2Py4),
2,4-Di-(2-pyridyl)-3,7-dimethyl-3,7-diazabicyclo[3.3.1]nonan-9-on-1,5-dimethyl-dicarboxylat (N2Py2),
2,4-Di-(2-pyridyl)-3,7-dimethyl-3,7-diazabicyclo[3.3.1]nonan-9-on-1,5-diethyl-dicarboxylat,
2,4-Di-(2-pyridyl)-3-methyl-7-(N,N'-dimethylethylamin)-3,7-diazabicyclo[3.3.1]nonan-9-on-1,5-dimethyl-dicarboxylat
und den entsprechenden Dihydroxyketalen.

In einer hierunter wiederum bevorzugten Ausführungsform der Erfindung werden mit dem erfindungsgemäßen Verfahren ein oder mehrere Komplexe der Formel [FeLCl]Cl, [FeL(SO₄)], [MnLCl]Ck oder [MnL(SO₄)], besonders bevorzugt ein oder mehrere Komplexe der Formel [FeLCl]Cl oder [FeL(SO₄)] und insbesondere bevorzugt ein oder mehrere Komplexe der Formel [FeLCl]Cl hergestellt.

Die in das erfindungsgemäße Verfahren eingesetzten Liganden der allgemeinen Formel (1) werden durch das Verfahren komplexiert und finden sich somit in den hergestellten Metallkomplexen der allgemeinen Formel (2) wieder. Allerdings können sie in den Metallkomplexen der allgemeinen Formel (2) derart modifiziert vorliegen, dass eine in den Ausgangsliganden der allgemeinen Formel (1) enthaltene Keton- oder Carbonylgruppe z (z = C=O) durch die Anwesenheit von Wasser während des erfindungsgemäßen Verfahrens in die hydratisierte Form überführt wird (z = C(OH)₂). Dies bedeutet, dass die Liganden in den Metallkomplexen der allgemeinen Formel (2) als Dihydroxyketale vorliegen können, auch wenn sie in Form der Ketone in das erfindungsgemäße Verfahren eingesetzt worden sind.

Dass die Liganden oftmals in komplexierter Form als Dihydroxyketale (z = C(OH)₂) vorliegen, wird z. B. in Inorg. Chimica Acta, 337 (2002) 407- 419 durch Röntgenstrukturanalyse gezeigt.

Die Herstellung der Liganden im großtechnischen Maßstab kann gemäß den Angaben in DE 601 20 781 oder WO 2006/133869 nach folgendem Reaktionsschema erfolgen:

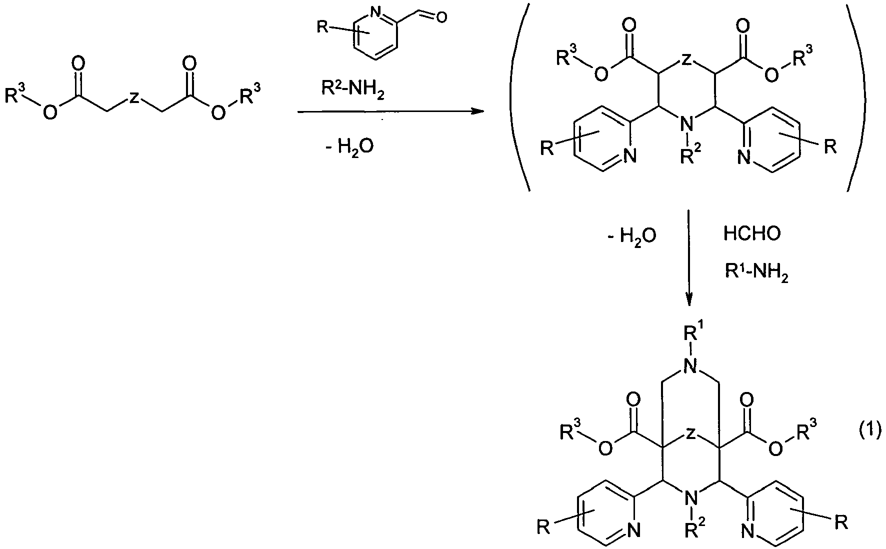

Ausgehend von Dicarbonsäurediester werden in einem C₁-C₄-Alkohol, beispielsweise Ethanol, Propanolen oder Butanolen, zwei Mannich Kondensationsschritte unter Wasserabspaltung durchgeführt. Nach beendeter Wasserabtrennung wird abgekühlt, das Produkt abfiltriert und gewaschen. Je nach Herstellung können die Liganden in Form mehr oder weniger großer Kristalle anfallen. Für die Komplexierungsreaktion können sie dann entweder lösemittelfeucht oder in getrockneter Form eingesetzt werden. Obwohl es für die Komplexierungsreaktion vorteilhaft sein sollte, möglichst kleine Kristalle in der heterogenen Komplexierungsreaktion einzusetzen, ist eine Zerkleinerung nicht unbedingt notwendig, damit die Umsetzung zum Erfolg führt.

In einer weiteren bevorzugten Ausführungsform des erfindungsgemäßen Verfahrens sind die Liganden der allgemeinen Formel (1) ausgewählt aus der Gruppe bestehend aus 2,4-Di-(2-pyridyl)-3-methyl-7-(pyridin-2-ylmethyl)-3,7-diazabicyclo[3.3.1]nonan-9-on-1,5-dimethyl-dicarboxylat (N2Py3o), 2,4-Di-(2-pyridyl)-3,7-dimethyl-3,7-diazabicyclo [3.3.1]nonan-9-on-1,5-dimethyl-dicarboxylat (N2Py2) und den entsprechenden Dihydroxyketalen.

Im Folgenden wird das in dem erfindungsgemäßen Verfahren verwendete Eisen- oder Mangansalz auch kurz als "Metallsalz" bezeichnet.

Das erfindungsgemäße Verfahren besteht ganz allgemein darin, dass der eine oder die mehreren Liganden in Wasser suspendiert werden und mit einem Metallsalz komplexiert werden.

Koordinierende Verbindungen X der Metallkomplexe der allgemeinen Formel (2) stammen vorzugsweise aus dem in dem erfindungsgemäßen Verfahren verwendeten Eisen- oder Mangansalz. Sie können aber z. B. auch aus dem Lösungsmittel stammen, insbesondere wenn X = H₂O ist.

Insbesondere bevorzugt sind koordinierende Verbindungen X ausgewählt aus der Gruppe bestehend aus Cl⁻ und SO₄²⁻. Hierunter bevorzugt ist Cl⁻.

Auch nicht koordinierende Gegenionen Y können vorzugsweise aus dem in dem erfindungsgemäßen Verfahren verwendeten Eisen- oder Mangansalz stammen, beispielsweise wenn Y die gleiche Bedeutung wie X besitzt.

Insbesondere bevorzugt sind nicht koordinierende Gegenionen Y ausgewählt aus der Gruppe bestehend aus Cl⁻ und SO₄²⁻. Hierunter bevorzugt ist Cl⁻.

In einer bevorzugten Ausführungsform der Erfindung haben X und Y die gleiche Bedeutung.

Vorzugsweise wird als Metallsalz ein Metall(II)-salz für das erfindungsgemäße Verfahren verwendet. In einer hierunter bevorzugten Ausführungsform der Erfindung ist das Metall(II)-salz ein Eisen(II)-salz, wobei Eisen(II)-chlorid und Eisen(II)-sulfat besonders bevorzugt sind. In einer weiteren hierunter bevorzugten Ausführungsform der Erfindung ist das Metall(II)-salz ausgewählt aus der Gruppe bestehend aus Eisen(II)-chlorid, Eisen(II)-sulfat, Mangan(II)-chlorid und Mangan(II)-sulfat. Als Metallsalz ist Eisen(II)-chlorid insbesondere bevorzugt.

In einer besonders bevorzugten Ausführungsform des erfindungsgemäßen Verfahrens werden ein oder mehrere Komplexe der Formel [FeLCl]Cl hergestellt, worin L 2,4-Di-(2-pyridyl)-3-methyl-7-(pyridin-2-ylmethyl)-3,7-diazabicyclo[3.3.1]nonan-9-on-1,5-dimethyl-dicarboxylat (N2Py3o) oder das entsprechende Dihydroxyketal bedeutet. Hierbei werden 2,4-Di-(2-pyridyl)-3-methyl-7-(pyridin-2-ylmethyl)-3,7-diazabicyclo[3.3.1]nonan-9-on-1,5-dimethyl-dicarboxylat oder das entsprechende Dihydroxyketal oder Mischungen davon mit Eisen(II)-chlorid umgesetzt.

In einer weiteren bevorzugten Ausführungsform des erfindungsgemäßen Verfahrens werden die Liganden L in Form der Ketone (z = C=O) in dem Verfahren eingesetzt.

In einer weiteren bevorzugten Ausführungsform des erfindungsgemäßen Verfahrens werden ein oder mehrere Metallkomplexe der allgemeinen Formel (2) hergestellt, worin die komplexierten Liganden L in Form der Dihydroxyketale (z = C(OH)₂) vorliegen.

Die Synthese erfolgt in der Weise, dass der eine oder die mehreren Liganden der Formel (1) in Wasser suspendiert werden und unter Rühren in einer heterogenen Reaktion mit einem Metallsalz in fester oder gelöster Form versetzt werden. Das Gewichtsverhältnis Wasser: Ligand beträgt dabei vorzugsweise von 4 : 1 bis 1 : 1, besonders bevorzugt von 2 : 1 bis 1,2 : 1. Das Molverhältnis
Ligand : Metallsalz beträgt vorzugsweise von 0,9 : 1 bis 1,2 : 1, besonders bevorzugt werden pro 1 Mol Ligand 0,99 bis 1,03 Mol Metallsalz verwendet. Das Metallsalz kann in fester Form entweder wasserfrei, bevorzugt jedoch in Hydratform (z. B. als Tetrahydrat), besonders bevorzugt jedoch in wässriger Lösung in einer Konzentration von 10 bis 50 Gew.-%, vorzugsweise jedoch von 20 bis 40 Gew.-%, eingesetzt werden.

Bei Zugabe des Metallsalzes zu den suspendierten Liganden tritt eine leichte Wärmetönung auf, die Reaktion bleibt jedoch über die gesamte Reaktionszeit hin heterogen. Der Umsatz der Reaktion wird analytisch (z. B. über HPLC) verfolgt.

Die Abtrennung der Metallkomplexe der Formel (2) kann als Feststoff aus dem Reaktionsgemisch gemäß den dem Fachmann geläufigen Methoden erfolgen und erfolgt vorzugsweise durch Filtration. Vorzugsweise wird der Komplex anschließend getrocknet, wobei er vor dem Trocknen vorzugsweise auch gewaschen werden kann.

Überraschenderweise wurde gefunden, dass die Temperatur und der pH-Wert während der Metallsalz-Zugabe und der Nachrührzeit einen entscheidenden Einfluss auf die Reaktionszeiten sowie Filtrationseigenschaften und damit auf die Ausbeute und Reinheit ausüben. Der pH-Wert des Reaktionsgemisches nach Zugabe des Metallsalzes (in fester oder gelöster Form) beträgt von 1 bis 3 und vorzugsweise von 1,5 bis 2,5. Der pH-Wert kann dabei durch Zugabe einer Säure zu den suspendierten Liganden oder über den pH-Wert der Metallsalzlösung eingestellt werden.

Wird die Reaktion bei Temperaturen unterhalb 30 °C durchgeführt, entsteht eine strukturviskose Suspension eines feinkristallinen Komplexes, dessen Isolierung mittels konventioneller Filterapparaturen kaum möglich ist, da es zur Blockierung des Filters und damit zu extrem langen Filtrationszeiten kommt. Die schlechte Filtrierbarkeit erschwert das Waschen und damit die Reinigung des Komplexes von überschüssigen Metallspuren bzw. nicht umgesetzten Liganden. Durch den hohen Feuchtigkeitsgehalt im Filterkuchen ergeben sich extrem lange Trocknungszeiten, wobei im Filterkuchen verbleibende Mutterlauge zum Verkleben des getrockneten Filterkuchens führt, so dass eine anschließende Mahlung unumgänglich wird, um ein pulverförmiges Endprodukt zu erhalten. Wird die Reaktion oberhalb 50 °C durchgeführt, beginnen sich die Liganden der Formel (1) unter den sauren Bedingungen der Reaktionslösung zu zersetzen, wodurch die Ausbeute an Endprodukt drastisch reduziert wird. Als mögliche Reaktionen könnten u. a. Hydrolyse der Esterbindung oder Retro-Mannich-Reaktionen in Frage kommen.

In einer bevorzugten Ausführungsform des erfindungsgemäßen Verfahrens wird die heterogene Komplexierungsreaktion deshalb bei Temperaturen von 40 bis 46 °C und besonders bevorzugt von 42 bis 45 °C, und einem pH von 1,5 bis 2,5 durchgeführt. Auf diese Weise erhält man in hoher Ausbeute kristalline Metallkomplexe mit sehr guten Filtrationseigenschaften und niederen Restfeuchten im Filterkuchen. Nach Trocknung erhält man ein frei fließendes Pulver, auf dessen Mahlung verzichtet werden kann.

In einer weiteren bevorzugten Ausführungsform der Erfindung haben X und Y eine unterschiedliche Bedeutung. In diesem Fall können z. B. zunächst Metallkomplexe der allgemeinen Formel (2), in denen X und Y die gleiche Bedeutung besitzen und besonders bevorzugt Chlorid bedeuten, hergestellt und anschließend die nicht koordinierenden Gegenionen Y ausgetauscht werden. Bei dieser Vorgehensweise wird zum Austausch von Y vorzugsweise ein Alkali- oder Erdalkalisalz, welches das neue nicht koordinierende Gegenion Y enthält, verwendet. Beispielsweise können Metallkomplexe der allgemeinen Formel (2) mit Y = PF₆⁻
(Hexafluorophosphate) erhalten werden, indem zunächst Metallkomplexe mit X = Y = Cl⁻ hergestellt werden und das nicht koordinierende Gegenion Cl⁻ anschließend mittels KPF₆ durch das neue nicht koordinierende Gegenion PF₆⁻ ausgetauscht wird. Derartige Austauschreaktionen sind dem Fachmann allgemein bekannt.

Im Vergleich zu den Verfahren gemäß dem Stand der Technik erzielt man bei dem erfindungsgemäßen Verfahren eine höhere Raum-Zeit-Ausbeute, kurze Filtrierzeiten und hohe Produktreinheiten. Der Verzicht auf organische Lösungsmittel erlaubt zudem einen kostengünstigen Herstellprozess. Nachfolgende Beispiele sollen die Erfindung näher erläutern ohne sie darauf einzuschränken.

### Beispiele

Herstellung des Liganden 2,4-Di-(2-pyridyl)-3-methyl-7-(pyridin-2-ylmethyl)-3,7-diazabicyclo[3.3.1]nonan-9-on-1,5-dimethyl-dicarboxylat (N2Py3o)

11,2 kg Acetondicarbonsäuredimethylester (97 Gew.-%ig; 64 mol) werden in 15 kg iso-Butanol gelöst. Die Lösung wird auf 10 °C abgekühlt, anschließend 13,4 kg Pyridin-2-aldehyd (99 Gew.-%ig, 125 mol) in 10 kg iso-Butanol, gefolgt von 4,8 kg Methylamin (40 Gew.-% in Wasser, 62 mol) so zugetropft, dass die Temperatur bei gleich bleibender Kühlung gehalten wird. Die Reaktionsmischung wird dann auf 40 - 45 °C erwärmt und im Vakuum bei 40 - 45 °C Innentemperatur ein Azeotrop (17 Liter) aus iso-Butanol und Wasser abdestilliert. Währenddessen werden 15 Liter iso-Butanol kontinuierlich zudosiert. Nach Abkühlung auf Raumtemperatur werden 8,4 kg Aminomethylpyridin (78 mol) zudosiert und der Dosiertrichter mit 7,0 kg iso-Butanol nachgewaschen. Dann werden 13,5 kg Formaldehydlösung (37 Gew.-% in Wasser, 166,5 mol) innerhalb von 15 - 30 Minuten zugegeben. Nach beendeter Zugabe wird die Mischung auf 55 - 60 °C erwärmt und 1,5 Stunden nachgerührt. Anschließend werden bei maximal 60 °C Innentemperatur 55 kg azeotropes Gemisch aus iso-Butanol und Wasser abdestilliert, während 36 kg iso-Butanol kontinuierlich zugegeben werden. Es wird mit Stickstoff belüftet und auf Raumtemperatur abgekühlt. Der gebildete Niederschlag wird abfiltriert und mit iso-Butanol gewaschen. Der Ligand kann in Form des feuchten Filterkuchens in die Komplexierungsreaktion eingesetzt werden oder aber im Vakuum bei 50 °C getrocknet werden. Dabei werden 23,3 kg (72,1 %) 2,4-Di-(pyridyl)-3-methyl-7-(pyridin-2-ylmethyl)-3,7-diazabicyclo[3.3.1]nonan-9-on-1,5-dimethyl-dicarboxylat in Form eines farblosen, kristallinen Pulvers erhalten.

### Beispiel 1

In einen Reaktionskessel wurden 220,0 kg (12,2 mol) Wasser und 145,1 kg (280 mol) 2,4-di-(2-pyridyl)-3-methyl-7-(pyridin-2-ylmethyl)-3,7-diazabicyclo[3.3.1]nonan-9-one-1,5-dimethyl-dicarboxylate (N2Py3o), Reinheit 99,5 %, eingefüllt und unter Rühren eine homogene Suspension hergestellt. Anschließend werden 119,5 kg (283 mol) wässrige Eisen(II)-chloridlösung (30,2 Gew.-%ig) innerhalb von 120 Minuten zugegeben. Nach 30 minütiger Zugabe beträgt der pH-Wert des Reaktionsgemisches ca. 1,8. Während dieser Zeit wird das Reaktionsgemisch auf 42 bis 45 °C erwärmt. Der Verlauf der Reaktion wird mittels HPLC-Messung verfolgt. Die Reaktionslösung wird 8 Stunden bei 42 bis 45 °C nachgerührt wobei der pH-Wert auf 2 ansteigt. Messungen der Korngrößenverteilung des gebildeten Komplexes der Formel (2) ergeben einen Durchschnittswert von 50 bis 70 µm. Anschließend wird über eine Nutsche abfiltriert. Aufgrund der guten Filtrationseigenschaften wird nach 30 minütiger Filtrationszeit ein Filterkuchen mit 25 Gew.-% Restfeuchte erhalten, der anschließend im Wärmeschrank bei 50 °C innerhalb 24 Stunden getrocknet wird, um eine Restfeuchte von < 1 Gew.-% zu erreichen. Man erhält auf diese Weise Eisen (1+), chloro[dimethyl 9,9-dihydroxy-3-methyl-2,4-di (2-pyridinyl- κN)-7-[(2-pyridinyl-κN)methyl]-3,7-diazabicyclo[3.3.1]nonane-1,5-dicarboxylate-κN3, κn7]-, chlorid (1:1) als gelbes fließfähiges Pulver.
Ausbeute (telquel): 97,7 %; Reinheit: 98,5 %; Ausbeute: 96,2 %

### Vergleichsbeispiel 1

Es wird analog Beispiel 1 verfahren, jedoch wird die Reaktion im Temperaturbereich von 20 bis 24 °C und einem Start-pH (30 Minuten nach Beginn der Zutropfzeit der Eisen(II)chlorid-Lösung) von 5,5 durchgeführt. Der Verlauf der Reaktion wird mittels HPLC-Messung verfolgt. Die Reaktionslösung muss 25 Stunden bei 20 bis 24 °C gerührt werden, um die Reaktion zu vervollständigen, wobei der pH-Wert auf 1,7 abfällt. Messungen der Korngrößenverteilung des gebildeten Komplexes der Formel (2) ergeben einen Durchschnittswert von 18 bis 30 µm. Anschließend wird über eine Nutsche abfiltriert. Aufgrund der schlechten Filtrationseigenschaften wird nach 160 minütiger Filtrationszeit ein Filterkuchen mit 44 Gew.-% Restfeuchte erhalten, der anschließend im Wärmeschrank bei 50 °C innerhalb 125 Stunden getrocknet wird, um eine Restfeuchte von
< 1 Gew.-% zu erreichen. Man erhielt auf diese Weise Eisen (1+), chloro[dimethyl 9,9-dihydroxy-3-methyl-2,4-di (2-pyridinyl-κN)-7-[(2-pyridinyl-κN)methyl]-3,7-diazabicyclo[3.3.1]nonane-1,5-dicarboxylate-κN3, κn7]-, chlorid (1:1) als gelbbraunen verbackenen Feststoff, der vor der weiteren Verarbeitung aufgemahlen werden muss. Ausbeute (telquel): 96,7 %; Reinheit: 95,4 %; Ausbeute: 92,2 %

Dieses Vergleichsbeispiel zeigt, dass durch die Reaktionsbedingungen, die nicht denen des erfindungsgemäßen Verfahrens entsprechen, die Reaktionszeit verdreifacht sowie die Filtrationszeit und die Trockenzeit verfünffacht werden und eine Mahlung des Endproduktes unbedingt erforderlich ist.

### Beispiel 2 und Vergleichsbeispiele V2 und V3

### Allgemeine Vorschrift

Im 1 Liter-5-Halskolben werden 155,4 g (0,3 mol) N2Py3o (99,5 Gew.-%ig) und 240 g von 247,8 g entsalztem Wasser vorgelegt. Der Ansatz wird 1 Stunde bei Raumtemperatur suspendiert. Unter Rühren wird in 1 Stunde 126,8 g (0,3 mol) FeCl₂-Lösung (30,0 Gew.-%ig) zugetropft. Der Ansatz wird 3 Stunden nachgerührt. Die Suspension wird über eine Nutsche abfiltriert, gewaschen und bei 50 °C unter N₂-Überlagerung getrocknet.

| | | V2 | V3 | Beispiel 2 |
|---|---|---|---|---|
| Zutropftemperatur | [°C] | 25-30 | 25-30 | 30-40 |
| Nachrührtemperatur | [°C] | 25-30 | 50-52 | 42-45 |
| pH nach Zugabe der FeCl₂-Lösung | | 3,2 | 3,2 | 2,0 |
| End-pH | | 3,3 | 1,7 | 1,9 |
| Partikelgröße | [µm] | 18 | 54 | 67 |
| Filtrierbarkeit | | schlecht | gut | gut |
| Restfeuchte im Filterkuchen | [Gew.-%] | 35 | 18 | 15 |
| Ausbeute (telquel) | [%] | 96,4 | 88,0 | 97,6 |
| Reinheit | [%] | 94 | 98 | 98 |
| Ausbeute | [%] | 91 | 86 | 96 |
| Produktform | | Brocken | Pulver | Pulver |

## Patentansprüche

1. Verfahren zur Herstellung eines oder mehrerer Metallkomplexe der allgemeinen Formel (2)
[MₐLₓXₙ]Yₘ (2)
wobei
M ein Metall aus der Gruppe Mn(II), Mn(III), Mn(IV), Fe(II), Fe(III) oder Fe(IV) ist,
X eine koordinierende Verbindung ausgewählt aus mono-, bi- oder trigeladenen Anionen oder neutralen Molekülen ist, die an ein Metall mono-, bi- oder tridentat koordinieren können,
Y ein nicht koordinierendes Gegenion darstellt, welches den Ladungsausgleich des Komplexes gewährleistet,
a eine Zahl von 1 bis 2 ist,
x eine Zahl von 1 bis 2 ist,
n eine Zahl von 0 bis 4 ist,
m eine Zahl von 0 bis 8 ist, und
L einen Liganden der allgemeinen Formel (1) oder seine protonierte oder deprotonierte Form 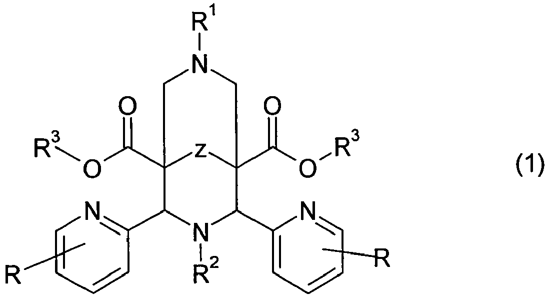
darstellt, wobei
R Wasserstoff, Hydroxyl oder C₁-C₄ Alkyl ist;
R¹ C₁-C₄ Alkyl, C₆-C₁₀ Aryl, Pyridinyl-C₁-C₄-alkyl oder (CH₂)ₖN(C₁-C₄-alkyl)₂ ist;
R² C₁-C₂₀ Alkyl, C₆-C₁₀ Aryl oder Pyridinyl-C₁-C₄-alkyl ist;
R³ C₁-C₄ Alkyl ist;
z C=O oder C(OH)₂ ist und
k eine Zahl von 1 bis 6 bedeutet,
**dadurch gekennzeichnet, dass** die Umsetzung eines oder mehrerer Liganden der Formel (1) mit einem Eisen- oder Mangansalz in heterogener Reaktion in Wasser durchgeführt wird, wobei die Reaktion in einem Temperaturbereich von 35 bis 50 °C durchgeführt wird und der pH-Wert des Reaktionsgemisches nach Zugabe des Eisen- oder Mangansalzes von 1 bis 3 beträgt.

2. Verfahren nach Anspruch 1, **dadurch gekennzeichnet, dass** X ausgewählt ist aus der Gruppe bestehend aus OH⁻, NO₃⁻, NO, S²⁻, R^{a}S⁻, PO₄³⁻, H₂O, CO₃²⁻, R^{b}OH, Cl⁻, Br⁻, CN⁻, ClO₄⁻, R^{a}COO⁻ und SO₄²⁻, wobei R^{a} H oder C₁-C₄ Alkyl und R^{b} C₁-C₄ Alkyl ist.

3. Verfahren nach Anspruch 1 oder 2, **dadurch gekennzeichnet, dass** Y ausgewählt ist aus der Gruppe bestehend aus R^{c}SO₄⁻, SO₄²⁻, NO₃⁻, Cl⁻, Br⁻, I⁻, ClO₄⁻, BF₄⁻, PF₆⁻ und R^{c}SO₃⁻, wobei R^{c} H oder C₁-C₄ Alkyl ist.

4. Verfahren nach einem oder mehreren der Ansprüche 1 bis 3, **dadurch gekennzeichnet, dass** die Reaktion in einem Temperaturbereich von 40 bis 46 °C durchgeführt wird.

5. Verfahren nach einem oder mehreren der Ansprüche 1 bis 4, **dadurch gekennzeichnet, dass** der pH-Wert des Reaktionsgemisches nach Zugabe des Eisen- oder Mangansalzes von 1,5 bis 2,5 beträgt.

6. Verfahren nach einem oder mehreren der Ansprüche 1 bis 5, **dadurch gekennzeichnet, dass** ein oder mehrere Komplexe der Formel [FeLCl]Cl, [FeL(SO₄)], [MnLCl]Cl, [MnL(SO₄)], [FeLCl]PF₆, [FeL(H₂O)][PF₆]2 oder [FeL(H₂O)][BF₄]₂ hergestellt werden, wobei L ausgewählt ist aus der Gruppe bestehend aus
2,4-Di-(2-pyridyl)-3-methyl-7-(pyridin-2-ylmethyl)-3,7-diazabicyclo[3.3.1]nonan-9-on-1,5-dimethyl-dicarboxylat (N2Py3o),
2,4-Di-(2-pyridyl)-3-(pyridin-2-ylmethyl)-7-methyl-3,7-diazabicyclo[3.3.1]nonan-9-on-1,5-dimethyl-dicarboxylat (N2Py3u),
2,4-Di-(2-pyridyl)-3-methyl-7-(pyridin-2-ylmethyl)-3,7-diazabicyclo[3.3.1]nonan-9-on-1,5-diethyl-dicarboxylat,
2,4-Di-(2-pyridyl)-3,7-bis-(pyridin-2-ylmethyl)-3,7-diazabicyclo[3.3.1]nonan-9-on-1,5-dimethyl-dicarboxylat (N2Py4),
2,4-Di-(2-pyridyl)-3,7-dimethyl-3,7-diazabicyclo[3.3.1]nonan-9-on-1,5-dimethyl-dicarboxylat (N2Py2),
2,4-Di-(2-pyridyl)-3,7-dimethyl-3,7-diazabicyclo[3.3.1]nonan-9-on-1,5-diethyl-dicarboxylat,
2,4-Di-(2-pyridyl)-3-methyl-7-(N,N'-dimethylethylamin)-3,7-diazabicyclo[3.3.1]nonan-9-on-1,5-dimethyl-dicarboxylat
und den entsprechenden Dihydroxyketalen.

7. Verfahren nach einem oder mehreren der Ansprüche 1 bis 6, **dadurch gekennzeichnet, dass** die Liganden der allgemeinen Formel (1) ausgewählt sind aus der Gruppe bestehend aus 2,4-Di-(2-pyridyl)-3-methyl-7-(pyridin-2-ylmethyl)-3,7-diazabicyclo[3.3.1]nonan-9-on-1,5-dimethyl-dicarboxylat (N2Py3o), 2,4-Di-(2-pyridyl)-3,7-dimethyl-3,7-diazabicyclo[3.3.1] nonan-9-on-1,5-dimethyl-dicarboxylat (N2Py2) und den entsprechenden Dihydroxyketalen.

8. Verfahren nach Anspruch 2, **dadurch gekennzeichnet, dass** X ausgewählt ist aus der Gruppe bestehend aus Cl⁻ und SO₄²⁻ und vorzugsweise Cl⁻ ist.

9. Verfahren nach Anspruch 3, **dadurch gekennzeichnet, dass** Y ausgewählt ist aus der Gruppe bestehend aus Cl⁻ und SO₄²⁻ und vorzugsweise Cl⁻ ist.

10. Verfahren nach einem oder mehreren der Ansprüche 1 bis 9, **dadurch gekennzeichnet, dass** das eingesetzte Eisen- oder Mangansalz ein Metall(II)-salz ist.

11. Verfahren nach Anspruch 10, **dadurch gekennzeichnet, dass** das Metall(II)-salz ausgewählt ist aus der Gruppe bestehend aus Eisen(II)-chlorid, Eisen(II)-sulfat, Mangan(II)-chlorid und Mangan(II)-sulfat.

12. Verfahren nach Anspruch 11, **dadurch gekennzeichnet, dass** das Metall(II)-salz Eisen(II)-chlorid ist.

13. Verfahren nach einem oder mehreren der Ansprüche 1 bis 12, **dadurch gekennzeichnet, dass** ein oder mehrere Komplexe der Formel [FeLCl]Cl hergestellt werden, worin L 2,4-Di-(2-pyridyl)-3-methyl-7-(pyridin-2-ylmethyl)-3,7-diazabicyclo[3.3.1]nonan-9-on-1,5-dimethyl-dicarboxylat (N2Py3o) oder das entsprechende Dihydroxyketal bedeutet, und 2,4-Di-(2-pyridyl)-3-methyl-7-(pyridin-2-ylmethyl)-3,7-diazabicyclo[3.3.1] nonan-9-on-1,5-dimethyl-dicarboxylat oder das entsprechende Dihydroxyketal oder Mischungen davon mit Eisen(II)-chlorid umgesetzt werden.

14. Verfahren nach einem oder mehreren der Ansprüche 1 bis 13, **dadurch gekennzeichnet, dass** die Liganden L in Form der Ketone (z = C=O) in dem Verfahren eingesetzt werden.

15. Verfahren nach einem oder mehreren der Ansprüche 1 bis 14, **dadurch gekennzeichnet, dass** ein oder mehrere Metallkomplexe der allgemeinen Formel (2) hergestellt werden, worin die komplexierten Liganden L in Form der Dihydroxyketale (z = C(OH)₂) vorliegen.

16. Verfahren nach einem oder mehreren der Ansprüche 1 bis 15, **dadurch gekennzeichnet, dass** die heterogene Komplexierungsreaktion bei Temperaturen von 42 bis 45 °C und einem pH-Wert von 1,5 bis 2,5 durchgeführt wird.

## Claims

1. A process for preparing one or more metal complexes of the general formula (2)
[MₐLₓXₙ]Yₘ (2)
where
M is a metal from the group of Mn(II), Mn(III), Mn(IV), Fe(II), Fe(III) or Fe(IV),
X is a coordinating compound selected from singly, doubly or triply charged anions or uncharged molecules capable of mono-, bi- or tridentate coordination to a metal,
Y is a noncoordinating counterion which ensures the charge balance of the complex,
a is a number from 1 to 2,
x is a number from 1 to 2,
n is a number from 0 to 4,
m is a number from 0 to 8, and
L is a ligand of the general formula (1) or the protonated or deprotonated form thereof 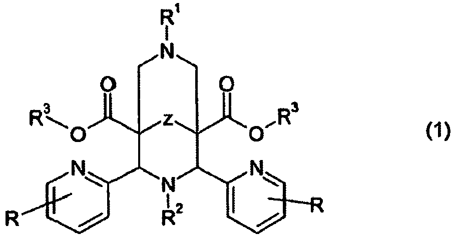
where
R is hydrogen, hydroxyl or C₁-C₄ alkyl;
R¹ is C₁-C₄ alkyl, C₆-C₁₀ aryl, pyridinyl-C₁-C₄-alkyl or (CH₂)ₖN(C₁-C₄-alkyl)₂;
R² is C₁-C₂₀ alkyl, C₆-C₁₀ aryl or pyridinyl-C₁-C₄-alkyl;
R³ is C₁-C₄ alkyl;
z is C=O or C(OH)₂ and
k is a number from 1 to 6,
which comprises performing the reaction of one or more ligands of the formula (1) with an iron or manganese salt in heterogeneous reaction in water, the reaction being performed within a temperature range from 35 to 50°C and the pH of the reaction mixture after addition of the iron or manganese salt being from 1 to 3.

2. The process as claimed in claim 1, wherein X is selected from the group consisting of OH⁻, NO₃⁻, NO, S²⁻, R^{a}S⁻, PO₄³⁻, H₂O, CO₃²⁻, R^{b}OH, Cl⁻, Br⁻, CN⁻, ClO₄⁻, R^{a}COO⁻ and SO₄²⁻, where R^{a} is H or C₁-C₄ alkyl and R^{b} is C₁-C₄ alkyl.

3. The process as claimed in claim 1 or 2, wherein Y is selected from the group consisting of R^{c}SO₄⁻, SO₄²⁻, NO₃⁻, Cl⁻, Br⁻, I⁻, ClO₄⁻, BF₄⁻, PF₆⁻ and R^{c}SO₃⁻, where R^{c} is H or C₁-C₄ alkyl.

4. The process as claimed in one or more of claims 1 to 3, wherein the reaction is performed within a temperature range from 40 to 46°C.

5. The process as claimed in one or more of claims 1 to 4, wherein the pH of the reaction mixture after addition of the iron or manganese salt is from 1.5 to 2.5.

6. The process as claimed in one or more of claims 1 to 5, wherein one or more complexes of the formula [FeLCI]CI, [FeL(SO₄)], [MnLCI]CI, [MnL(SO₄)], [FeLCl]PF₆, [FeL(H₂O)][PF₆]2 or [FeL(H₂O)][BF₄]₂ are prepared, where L is selected from the group consisting of
dimethyl 2,4-di(2-pyridyl)-3-methyl-7-(pyridin-2-ylmethyl)-3,7-diaza-bicyclo[3.3.1]nonan-9-one-1,5-dicarboxylate (N2Py3o),
dimethyl 2,4-di(2-pyridyl)3-(pyridin-2-ylmethyl)-7-methyl-3,7-diaza-bicyclo[3.3.1]nonan-9-one-1,5-dicarboxylate (N2Py3u),
diethyl 2,4-di(2-pyridyl)-3-methyl-7-(pyridin-2-ylmethyl)-3,7-diaza-bicyclo[3.3.1]nonan-9-one-1,5-dicarboxylate,
dimethyl 2,4-di(2-pyridyl)-3,7-bis(pyridin-2-ylmethyl)-3,7-diaza-bicyclo[3.3.1]nonan-9-one-1,5-dicarboxylate (N2Py4),
dimethyl 2,4-di(2-pyridyl)-3,7-dimethyl-3,7-diazabicyclo[3.3.1]nonan-9-one-1,5-dicarboxylate (N2Py2),
diethyl 2,4-di(2-pyridyl)-3,7-dimethyl-3,7-diazabicyclo[3.3.1]nonan-9-one-1,5-dicarboxylate,
dimethyl 2,4-di(2-pyridyl)-3-methyl-7-(N,N'-dimethylethylamine)-3,7-diazabicyclo[3.3.1]nonan-9-one-1,5-dicarboxylate
and the corresponding dihydroxy ketals.

7. The process as claimed in one or more of claims 1 to 6, wherein the ligands of the general formula (1) are selected from the group consisting of dimethyl 2,4-di(2-pyridyl)-3-methyl-7-(pyridin-2-ylmethyl)-3,7-diazabicyclo[3.3.1]nonan-9-one-1,5-dicarboxylate (N2Py3o), dimethyl 2,4-di(2-pyridyl)-3,7-dimethyl-3,7-diazabicyclo-[3.3.1]nonan-9-one-1,5-dicarboxylate (N2Py2) and the corresponding dihydroxy ketals.

8. The process as claimed in claim 2, wherein X is selected from the group consisting of Cl⁻ and SO₄²⁻ and is preferably Cl⁻.

9. The process as claimed in claim 3, wherein Y is selected from the group consisting of Cl⁻ and SO₄²⁻ and is preferably Cl⁻.

10. The process as claimed in one or more of claims 1 to 9, wherein the iron or manganese salt used is a metal(II) salt.

11. The process as claimed in claim 10, wherein the metal(II) salt is selected from the group consisting of iron(II) chloride, iron(II) sulfate, manganese(II) chloride and manganese(II) sulfate.

12. The process as claimed in claim 11, wherein the metal(II) salt is iron(II) chloride.

13. The process as claimed in one or more of claims 1 to 12, wherein one or more complexes of the formula [FeLC]Cl are prepared in which L is dimethyl 2,4-di(2-pyridyl)-3-methyl-7-(pyridin-2-ylmethyl)-3,7-diazabicyclo[3.3.1]nonan-9-one-1,5-dicarboxylate (N2Py3o) or the corresponding dihydroxy ketal, and dimethyl 2,4-di(2-pyridyl)-3-methyl-7-(pyridin-2-ylmethyl)-3,7-diazabicyclo[3.3.1]-nonan-9-one-1,5-dicarboxylate or the corresponding dihydroxy ketal, or mixtures thereof, are reacted with iron(II) chloride.

14. The process as claimed in one or more of claims 1 to 13, wherein the ligands L are used in the process in the form of the ketones (z = C=O).

15. The process as claimed in one or more of claims 1 to 14, wherein one or more metal complexes of the general formula (2) are prepared in which the complexed ligands L are present in the form of the dihydroxy ketals (z = C(OH)₂).

16. The process as claimed in one or more of claims 1 to 15, wherein the heterogeneous complexation reaction is performed at temperatures of 42 to 45°C and a pH of 1.5 to 2.5.

## Revendications

1. Procédé pour la préparation d'un ou de plusieurs complexes métalliques de formule générale (2)
[MₐLₓXₙ]Yₘ (2)
dans laquelle
M représente un métal du groupe Mn(II), Mn(III), Mn(IV), Fe(II), Fe(III) ou Fe(IV),
X représente un composé coordinant choisi parmi les anions présentant une, deux ou trois charges ou les molécules neutres qui peuvent se coordiner à un métal monodentate, bidentate ou tridentate,
Y représente un contre-ion non coordinant qui assure l'équilibre des charges du complexe,
a est un nombre de 1 à 2,
x est un nombre de 1 à 2,
n est un nombre de 0 à 4,
m est un nombre de 0 à 8 et
L représente un ligand de formule générale (1) ou sa forme protonée ou déprotonée, 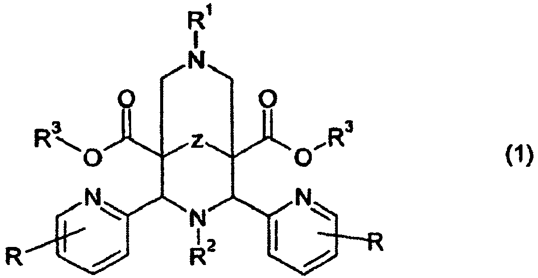
dans laquelle
R représente hydrogène, hydroxyle ou C₁-C₄-alkyle ;
R¹ représente C₁-C₄-alkyle, C₆-C₁₀-aryle, pyridinyl-C₁-C₄-alkyle ou (CH₂)ₖN(C₁-C₄-alkyle)₂ ;
R² représente C₁-C₂₀-alkyle, C₆-C₁₀-aryle ou pyridinyl-C₁-C₄-alkyle ;
R³ représente C₁-C₄-alkyle ;
z représente C=O ou C(OH)₂ et
k signifie un nombre de 1 à 6,
**caractérisé en ce que** la transformation d'un ou de plusieurs ligands de formule (1) avec un sel de fer ou de manganèse est réalisée dans une réaction hétérogène dans l'eau, la réaction étant réalisée dans une plage de température de 35 à 50°C et la valeur du pH du mélange réactionnel après addition du sel de fer ou de manganèse étant de 1 à 3.

2. Procédé selon la revendication 1, **caractérisé en ce que** X est choisi dans le groupe constitué par OH⁻, NO₃⁻, NO, S²⁻, R^{a}S⁻, PO₄³⁻, H₂O, CO₃²⁻, R^{b}OH, Cl⁻, Br⁻, CN⁻, ClO₄⁻, R^{a}COO⁻ et SO₄²⁻ ; R^{a} représentant H ou C₁-C₄-alkyle et R^{b} représentant C₁-C₄-alkyle.

3. Procédé selon la revendication 1 ou 2, **caractérisé en ce que** Y est choisi dans le groupe constitué par R^{c}SO₄⁻, SO₄²⁻, NO₃⁻, Cl⁻, Br⁻, I⁻, ClO₄⁻, BF₄⁻, PF₆⁻ et R^{c}SO₃⁻; R^{c} représentant H ou C₁-C₄-alkyle.

4. Procédé selon l'une ou plusieurs des revendications 1 à 3, **caractérisé en ce que** la réaction est réalisée dans une plage de température de 40 à 46°C.

5. Procédé selon l'une ou plusieurs des revendications 1 à 4, **caractérisé en ce que** la valeur du pH du mélange réactionnel après addition du sel de fer ou de manganèse est de 1,5 à 2,5.

6. Procédé selon l'une ou plusieurs des revendications 1 à 5, **caractérisé en ce qu'**un ou plusieurs complexes de formule [FeLCl]Cl, [FeL(SO₄)], [MnLCl]Cl, [MnL(SO₄)], [FeLCl]PF6, [FeL(H₂O)][PF₆]₂ ou [FeL(H₂O)][BF₄]₂ sont préparés, L étant choisi dans le groupe constitué par
1,5-diméthyldicarboxylate de 2,4-di-(2-pyridyl)-3-méthyl-7-(pyridin-2-ylméthyl)-3,7-diazabicyclo[3,3,1]nonan-9-one (N2Py3o),
1,5-diméthyldicarboxylate de 2,4-di-(2-pyridyl)-3-(pyridin-2-ylméthyl)-7-méthyl-3,7-diazabicyclo[3,3,1]nonan-9-one (N2Py3u),
1,5-diéthyldicarboxylate de 2,4-di-(2-pyridyl)-3-méthyl-7-(pyridin-2-ylméthyl)-3,7-diazabicyclo[3,3,1]nonan-9-one,
1,5-diméthyldicarboxylate de 2,4-di-(2-pyridyl)-3,7-bis-(pyridin-2-ylméthyl)-3,7-diazabicyclo[3,3,1]nonan-9-one (N2Py4),
1,5-diméthyldicarboxylate de 2,4-di-(2-pyridyl)-3,7-diméthyl-3,7-diazabicyclo[3,3,1]nonan-9-one (N2Py2),
1,5-diéthyldicarboxylate de 2,4-di-(2-pyridyl)-3,7-diméthyl-3,7-diazabicyclo[3,3,1]nonan-9-one,
1,5-diméthyldicarboxylate de 2,4-di-(2-pyridyl)-3-méthyl-7-(N,N'-diméthyléthylamino)-3,7-diazabicyclo[3,3,1]nonan-9-one
et les dihydroxycétals correspondants.

7. Procédé selon l'une ou plusieurs des revendications 1 à 6, **caractérisé en ce que** les ligands de formule générale (1) sont choisis dans le groupe constitué par le 1,5-diméthyldicarboxylate de 2,4-di-(2-pyridyl)-3-méthyl-7-(pyridin-2-ylméthyl)-3,7-diazabicyclo[3,3,1]nonan-9-one (N2Py3o), le 1,5-diméthyldicarboxylate de 2,4-di-(2-pyridyl)-3,7-diméthyl-3,7-diazabicyclo[3,3,1]nonan-9-one (N2Py2) et les dihydroxycétals correspondants.

8. Procédé selon la revendication 2, **caractérisé en ce que** X est choisi dans le groupe constitué par Cl⁻ et SO₄²⁻ et est de préférence Cl⁻.

9. Procédé selon la revendication 3, **caractérisé en ce que** Y est choisi dans le groupe constitué par Cl⁻ et SO₄²⁻ et est de préférence Cl⁻.

10. Procédé selon l'une ou plusieurs des revendications 1 à 9, **caractérisé en ce que** le sel de fer ou de manganèse utilisé est un sel de métal (II).

11. Procédé selon la revendication 10, **caractérisé en ce que** le sel de métal (II) est choisi dans le groupe constitué par le chlorure de fer (II), le sulfate de fer (II), le chlorure de manganèse (II) et le sulfate de manganèse (II).

12. Procédé selon la revendication 11, **caractérisé en ce que** le sel de métal (II) est le chlorure de fer (II).

13. Procédé selon l'une ou plusieurs des revendications 1 à 12, **caractérisé en ce qu'**un ou plusieurs complexes de formule [FeLCl]Cl sont préparés, dans laquelle L représente le 1,5-diméthyldicarboxylate de 2,4-di-(2-pyridyl)-3-méthyl-7-(pyridin-2-ylméthyl)-3,7-diazabicyclo[3,3,1]nonan-9-one (N2Py3o) ou le dihydroxycétal correspondant, et du 1,5-diméthyldicarboxylate de 2,4-di-(2-pyridyl)-3-méthyl-7-(pyridin-2-ylméthyl)-3,7-diazabicyclo[3,3,1]nonan-9-one ou le dihydroxycétal correspondant ou des mélanges de ceux-ci sont transformés avec du chlorure de fer (II).

14. Procédé selon l'une ou plusieurs des revendications 1 à 13, **caractérisé en ce que** les ligands L sont utilisés sous la forme des cétones (z = C=O) dans le procédé.

15. Procédé selon l'une ou plusieurs des revendications 1 à 14, **caractérisé en ce qu'**un ou plusieurs complexes métalliques de formule générale (2) sont préparés, dans laquelle les ligands complexés L se trouvent sous forme des dihydroxycétals (z = C(OH)₂).

16. Procédé selon l'une ou plusieurs des revendications 1 à 15, **caractérisé en ce que** la réaction de complexation hétérogène est réalisée à des températures de 42 à 45°C et à un pH de 1,5 à 2,5.
